# EUROPEAN PATENT APPLICATION

(11) **EP 4 512 387 A1**
(43) Date of publication of application: **26.02.2025**
(21) Application number: 23792190.3
(22) Date of filing: 20.04.2023
(51) Int. Cl.: A61K 8/04, A61K 8/73, A61K 8/42, A61Q 13/00

(54) **PERFUME GEL COMPOSITION**

(30) Priority: 21.04.2022 KR 20220049477
(71) Applicant: Jinyoungbio Co. Ltd, Seoul 03759 (KR)
(72) Inventor: PARK, Sung-Jin, Seongnam-si, Gyeonggi-do 13608 (KR); SHIN, Hyun Man, Seoul 08632 (KR); LEE, Soo Chul, Ansan-si, Gyeonggi-do 15632 (KR); CHOI, Woo Kyung, Anyang-si, Gyeonggi-do 13914 (KR); PARK, Yong Il, Hwaseong-si, Gyeonggi-do 18324 (KR); KIM, Sang Hyeon, Suwon-si, Gyeonggi-do 16598 (KR)
(74) Representative: Kraus & Lederer PartGmbB
(86) International application number: PCT/KR2023/005355
(87) International publication number: WO 2023/204616

(57) **Abstract**

Disclosed herein are a perfume gel composition and a preparation method therefor wherein the perfume composition contains an oil alone or a combination of oils and a dextrin-based oil gelling agent alone or in combination with an amino acid-based oil gelling agent, and employs a minimal amount of alcohol (ethanol) or no alcohols. The perfume gel composition using the oil gelling agent complements the disadvantages of the existing liquid type or liquid spray type perfume to improve the fragrance persistence and is advantageous in terms of ease of use and economy without dripping over the desired skin area or being lost. Furthermore, the composition can be housed in a variety of containers including pump-type, tube-type, regular lotion-type, or cream-type containers.

## Description

### [Technical Field]

A perfume composition prepared and used in a gel form is presented in the present specification. Specifically, since it is in the form of a gel, a perfume composition that may be applied to a desired area is presented.

### [Background Art]

The most commonly used formulation as a perfume is a liquid spray type. Such perfume formulations are generally sprayed over a wide area when used, and can even spray into the air other than the intended application area, causing damage and harming to others. In addition, since they are based on alcohol components such as ethanol, they are easily evaporated or have a short fragrance persistence, so they must be applied several times a day. The choice of containers is also limited, with liquid spray type containers being mainly used, and when used in the form of a liquid type applied like a skin toner, they flow down, causing inconvenience when used.

### [Detailed Description of the Invention]

### [Technical Problem]

In one aspect, the purpose of the present invention is to provide a perfume gel composition formulation that solves the disadvantages of the existing liquid spray form and is convenient to use.

In other aspect, the purpose of the present invention is to provide a perfume gel composition formulation that has a long-lasting fragrance.

In other aspect, the purpose of the present invention is to provide a perfume gel composition formulation that can use no or a minimal amount of alcohol such as ethanol.

In another aspect, the purpose of the present invention is to provide a perfume gel composition formulation based on an oil gel.

In another aspect, the purpose of the present invention is to provide a perfume gel composition formulation that enables topical application, that is, accurate and sufficient application even with a small amount appropriate for a desired area.

In another aspect, the purpose of the present invention is to provide a perfume composition of a skin application formulation that has a skin moisturizing effect and a soft feeling of use.

### [Solution to Problem]

In one aspect, the present invention relates to a perfume gel composition using an oil gelling agent, which is a composition comprising a dextrin-based oil gelling agent and/or an amino acid-based oil gelling agent as the oil gelling agent.

In one aspect, the perfume gel composition according to the present invention is a composition comprising a dextrin-based oil gelling agent and an amino acid-based oil gelling agent as the oil gelling agents.

In another aspect, the present invention relates to a method for producing the perfume gel composition according to the present invention, which may comprise adding the oil gelling agent to the oil and dissolving the mixture at a temperature of 60 to 160°C; and adding a fragrance to the dissolved substance.

### [Advantageous Effects of Invention]

In one aspect, the perfume gel composition using the oil gelling agent according to the present invention is applied only to the desired area in the form of a gel, and unlike the existing liquid spray type perfume, it does not disperse in the air, so there is no loss, and it does not cause damage to others.

In one aspect, the present invention is economical because it does not use or minimally uses alcohol such as ethanol, and is based on an oil gel, so the fragrance persists well.

In one aspect, the present invention is based on an oil gel, so the application area does not dry out, and it has a soft feeling of use.

### [Best Mode for Carrying Out the Invention]

The present invention relates to a perfume gel composition, wherein the perfume gel composition is a perfume gel composition based on an oil gel using an oil gelling agent, and is different from existing liquid perfumes based on an alcohol component (ethanol).

In one aspect, the perfume gel composition using an oil gelling agent according to the present invention may comprise a dextrin-based oil gelling agent and/or an amino acid-based oil gelling agent as the oil gelling agent. The dextrin-based oil gelling agent may be one that dissolves in oil at a temperature of 60°C or higher. The dextrin-based oil gelling agent may be a dextrin fatty acid ester.

In one embodiment, the fatty acid of the dextrin fatty acid ester may be an ester comprising a saturated fatty acid having 8 to 30 carbon atoms. For example, the carbon number of the fatty acid may be 8 or more, 10 or more, 12 or more, 14 or more, or 16 or more. In addition, the carbon number of the fatty acid may be 30 or less, 28 or less, 26 or less, 24 or less, 22 or less, 20 or less, 18 or less, or 16 or less. For example, the carbon number of the fatty acid may be 10 to 24, 12 to 20, 14 to 18, or 16. For example, it may be palmitic acid.

In one embodiment, the dextrin fatty acid ester may further comprise a substituted or unsubstituted C3-10 acid. For example, the substitution may be a substitution with a C1-6 alkyl. For example, the additional acid may be ethylhexanoic acid. In one embodiment, the dextrin fatty acid ester may be dextrin palmitate, dextrin palmitate/ethylhexanoate, or a mixture thereof.

The dextrin palmitate may have a structure as follows:

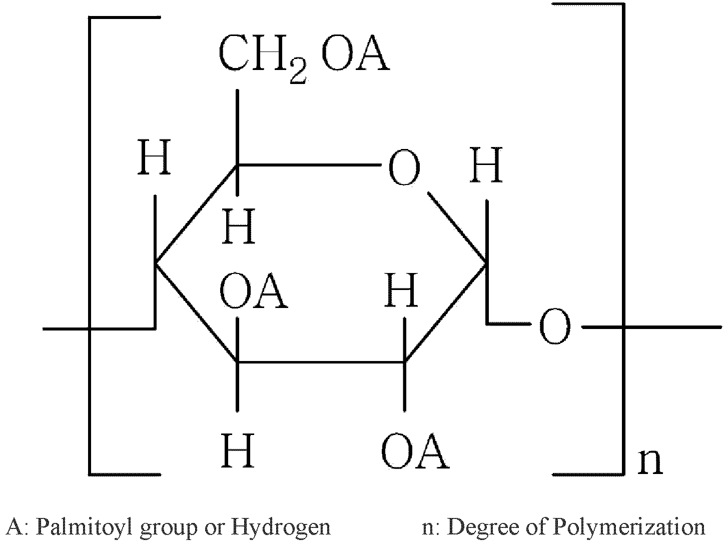

In one embodiment, the oil gelling agent may further comprise an amino acid-based oil gelling agent. In one embodiment, the amino acid-based oil gelling agent may be a glutamide-based gelling agent. In one embodiment, the glutamide-based gelling agent may be a dibutyl pentanediamide-based gelling agent. For example, it may be pentanediamide, N,N'-dibutyl-2-((1-oxoalkyl)amino)-, (2S)- [Pentanediamide, N,N'-dibutyl-2-((1-oxoalkyl)amino)-, (2S)-] according to the following Chemical Formula 1.

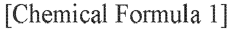

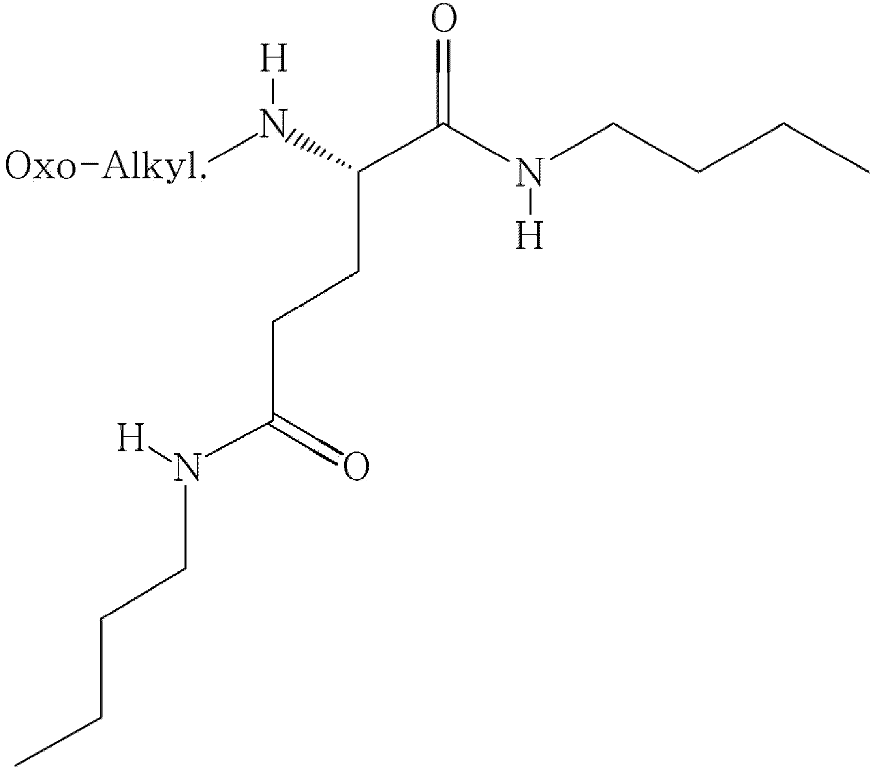

In the above formula, the alkyl of Oxo-Alkyl(Alkyl=O) may be an unsubstituted or substituted C4-16 alkyl. In addition, the substitution may be one in which one or more hydrogens are substituted with C1-6 alkyl. Here, the alkyl may be an unsubstituted or substituted C6-12. The substitution may be one in which one or more hydrogens are substituted with C1-4 alkyl.

For example, the gelling agent may be dibutylethylhexanoylglutamide [Pentanediamide, N,N'-dibutyl-2-[(2-ethyl-1-oxohexyl)amino]-, (2S)-] of the following Chemical Formula 2.

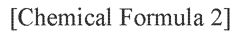

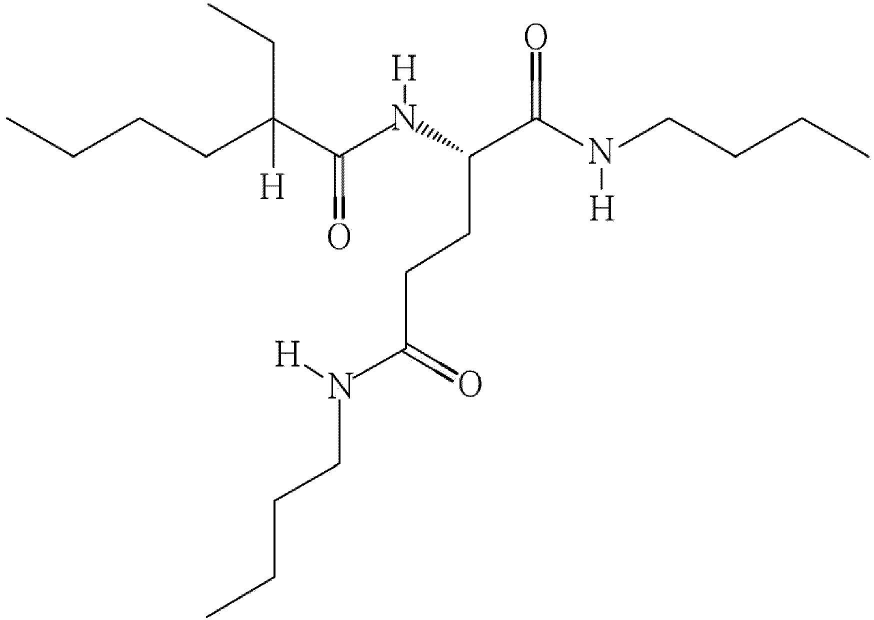

As another example, the gelling agent may be dibutyllauroylglutamide [Pentanediamide, N,N'-dibutyl-2-((1-oxododecyl)amino)-, (2S)-] of the following Chemical Formula 3.

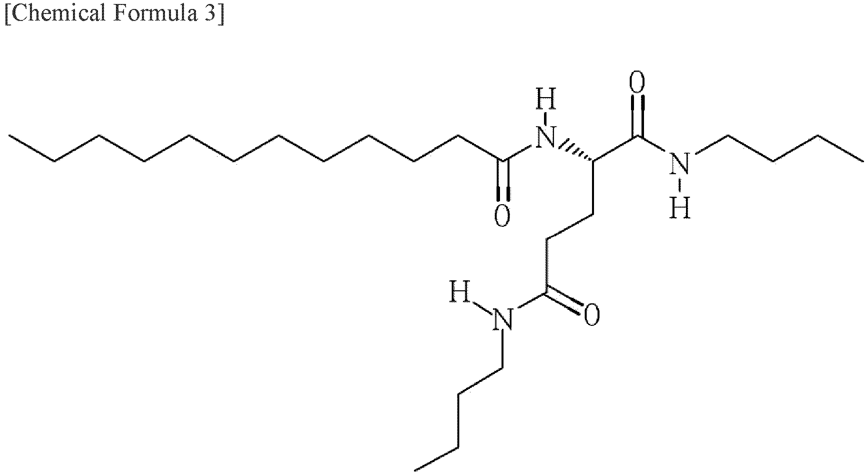

In one embodiment, the dibutyl pentanediamide-based gelling agent is pentanediamide, N,N'-dibutyl-2-((1-oxoalkyl)amino)-, (2S)- [Pentanediamide, N,N'-dibutyl-2-((1-oxoalkyl)amino)-, (2S)-], wherein the alkyl is unsubstituted or substituted C₄₋₁₆ alkyl, and the substitution may be a substitution of one or more hydrogens with C1-6 alkyl. For example, the dibutyl pentanediamide-based gelling agent may be dibutylethylhexanoylglutamide, dibutyllauroylglutamide, or a mixture thereof.

In one embodiment, the oil gelling agent may be contained in an amount of 0.01 to 30% by weight, based on the total weight of the composition. The ratio of the oil gelling agent may be adjusted according to desired characteristics, such as the degree of strength and weakness of the gel and the feeling of use. By setting the ratio of the oil gelling agent in various ways, various feelings of use may be simulated. By adjusting the ratio of the oil gelling agent and the oil, various perfume gels from soft gels to hard balm types may be implemented. In addition, since each oil has its own characteristics, oils may be used alone or in combination to implement even more various feelings of use.

In one embodiment, the dextrin-based oil gelling agent may be contained in an amount of 1 to 30% by weight, based on the total weight of the composition. For example, it may be 1 wt% or more, 2 wt% or more, 3 wt% or more, 4 wt% or more, 5 wt% or more, 6 wt% or more, 7 wt% or more, 8 wt% or more, 9 wt% or more, or 10 wt% or more. In addition, it may be 30 wt% or less, 28 wt% or less, 26 wt% or less, 24 wt% or less, 22 wt% or less, 20 wt% or less, 19 wt% or less, 18 wt% or less, 17 wt% or less, 16 wt% or less, 15 wt% or less, 14 wt% or less, or 13 wt% or less.

In one embodiment, the amino acid-based oil gelling agent may be 0.1 to 10% by weight, based on the total weight of the perfume gel composition. For example, it may be 0.1 wt% or more, 0.5 wt% or more, 1.0 wt% or more, 1.5 wt% or more, 2 wt% or more, 2.5 wt% or more, or 3.0 wt% or more. In addition, it may be 10 wt% or less, 9 wt% or less, 8 wt% or less, 7 wt% or less, 6 wt% or less, 5 wt% or less, 4 wt% or less, or 3 wt% or less.

In one embodiment, the weight ratio of the oil and the oil gelling agent may be from 2:1 to 99:1. For example, it may be 2:1 or greater, 3:1 or greater, 4:1 or greater, 5:1 or greater, 5.5:1 or greater, 5.8:1 or greater, 6:1 or greater, 7:1 or greater, 8:1 or greater, 9:1 or greater, 10:1 or greater, 20:1 or greater, 30:1 or greater, 40:1 or greater, 50:1 or greater, 60:1 or greater, 70:1 or greater, 80:1 or greater, or 90:1 or greater. Additionally, it may be 90:1 or less, 80:1 or less, 70:1 or less, 60:1 or less, 50:1 or less, 40:1 or less, 30:1 or less, 20:1 or less, 18:1 or less, 16:1 or less, 14:1 or less, 12:1 or less, 10:1 or less, 9:1 or less, 8:1 or less, 7:1 or less, or 6:1 or less.

The dextrin-based oil gelling agent may be used alone as the oil gelling agent, but a combination of the dextrin-based oil gelling agent and the amino acid-based oil gelling agent may also be used. In one embodiment, the weight ratio of the dextrin-based oil gelling agent and the amino acid-based oil gelling agent may be 100:0.1 to 50. Within this range, the oil gelling agent has a softer property on the skin and contributes to the fragrance persistence. For example, when the dextrin-based oil gelling agent is 100 parts by weight, the amino oil gelling agent may be 0.1 parts by weight or more, 1 part by weight or more, 10 parts by weight or more, 15 parts by weight or more, 20 parts by weight or more, or 25 parts by weight or more. Meanwhile, when the dextrin-based oil gelling agent is 100 parts by weight, the amino-based oil gelling agent may be 50 parts by weight or less, 45 parts by weight or less, 40 parts by weight or less, 35 parts by weight or less, 30 parts by weight or less, or 25 parts by weight or less.

In one specific example, the oil may be any oil except for silicone oil, castor oil, and fluorocarbons. Natural oils may also be used.

In one embodiment, the perfume gel composition may be substantially free of alcohol such as ethanol or water.

In one embodiment, the perfume gel composition using the oil gelling agent may be substantially free of wax.

In one embodiment, the perfume gel composition using the oil gelling agent may be substantially free of a silicone ingredient.

In the present specification, the term "substantially free of" means comprising in an amount of 5 wt% or less, 4 wt% or less, 3 wt% or less, 2 wt% or less, 1 wt% or less, 0.5 wt% or less, 0.2 wt% or less, 0.1 wt% or less, 0.01 wt% or less, 0.001 wt% or less, 0.0001 wt% or less, 0.00001 wt% or less, or 0.000001 wt% or less, based on the total composition.

In one embodiment, the perfume gel composition using the oil gelling agent may comprise a skin moisturizing ingredient or an ingredient helpful to a skin.

In one embodiment, the perfume gel composition may comprise the fragrance in an amount of 0.1 to 40% by weight, based on the total weight of the composition. For example, it may comprise 0.1 wt% or more, 0.5 wt% or more, 1 wt% or more, 5 wt% or more, 10 wt% or more, 15 wt% or more, 20 wt% or more, 25 wt% or more, or 30 wt% or more. **In** addition, it may comprise 40 wt% or less, 35 wt% or less, 30 wt% or less, 25 wt% or less, or 20 wt% or less.

In another aspect, the present invention relates to a method for producing the perfume gel composition using the oil gelling agent, which may comprise adding the oil gelling agent to the oil and dissolving the mixture at a temperature of 60 to 160°C.

In one embodiment, the step may be dissolving the mixture at a temperature of 60 to 160°C. for 10 to 20 minutes. Specifically, the temperature may be 60°C or higher, 65°C or higher, 70°C or higher, 75°C or higher, 80°C or higher, 85°C or higher, 90°C or higher, 95°C or higher, 100°C or higher, 110°C or higher, 115°C or higher, or 120°C or higher. In addition, the temperature may be 160°C or lower, 150°C or lower, 140°C or lower, 130°C or lower, 125°C or lower, 120°C or lower, 115°C or lower, 110°C or lower, 105°C or lower, 100°C or lower, or 95°C or lower. Meanwhile, the time may be 10 minutes or higher, 11 minutes or higher, 12 minutes or higher, 13 minutes or higher, 14 minutes or higher, 15 minutes or higher, 16 minutes or higher, 17 minutes or higher, or 18 minutes or higher. Additionally, the time may be 20 minutes or less, 19 minutes or less, 18 minutes or less, 17 minutes or less, 16 minutes or less, 15 minutes or less, 14 minutes or less, 13 minutes or less, 12 minutes or less, or 11 minutes or less.

In one embodiment, the method may further comprise adding a fragrance after the step of adding the oil gelling agent to the oil and dissolving it.

### [Mode for Carrying Out the Invention]

### Examples 1 to 5, Comparative Examples 1 to 2

According to the composition in Table 1 below, an oil gelling agent was added to the oil phase and dissolved by stirring at 1000 rpm with an agi mixer at a temperature of 120°C for 15 minutes. After that, the temperature was maintained at 90 to 100°C, and a fragrance was slowly added while stirring at 500 rpm or less with the agi mixer.

**[Table 1] Examples 1 to 5, and Comparative Example 1 to 2 for each oil gelling agent based on 30% fragrance**

| | Raw Material Name | Examp. 1 | Examp. 2 | Examp. 3 | Examp. 4 | Examp. 5 | Compar ative Examp. 1 | Compar ative Examp. 2 |
|---|---|---|---|---|---|---|---|---|
| Oil phase | Isononylisononate | 30 | 30 | 30 | 30 | 34 | | |
| | Cetylethylhexanoate | 29.8 | 29.8 | 29.8 | 29.8 | 34 | | |
| Water phase | Water | | | | | | 32.5 | |
| | Glycerin Ethanol | | | | | | 220 | |
| | Dipropyleneglycol | | | | | | 15 | |
| Oil gelling agent | Dextrin palmitate | 10.2 | | 5.1 | 5 | | | |
| | Dextrin palmitate / ethylhexanoate | | 10.2 | 5.1 | 5 | | | |
| | Dibutylethylhexanoylglu tamide | | | | 0.1 | 1 | | |
| | Dibutyllauroylglutamide | | | | 0.1 | 1 | | |
| | Acrylate/C10-30 alkyl acrylate crosspolymer | | | | | | 0.5 | |
| Fragran ce | Fragrance | 30 | 30 | 30 | 30 | 30 | 30 | |
| Compa rison | Perfume fragrance (Fragrance 30%) | | | | | | | 100 |

As shown in the above Table 1, Examples 1 to 4 of the present invention are oil gel compositions. Examples 1 to 3 of the present invention are compositions using dextrin palmitate and dextrin palmitate/ethylhexanoate, which are dextrin gelling agents, as oil gelling agents, respectively or in equal amounts at a ratio of 1:1. Example 4 is an example in which dextrin palmitate and dextrin palmitate/ethylhexanoate were used in a 1:1 ratio as in Example 3 as an oil gelling agent, and the amino acid-based gelling agent was added, and a composition that the total content of the oil gelling agent was the same as in Examples 1 to 3. Example 5 used only the amino acid-based gelling agent as the oil gelling agent, but since amino acid-based gelling agents have different gel-forming abilities and characteristics from dextrin-based gelling agents, the total content of the oil gelling agent was set as an exception only in Example 5. Comparative Example 1 is an example in which an aqueous gel containing ethanol is used as a base, unlike Examples 1 to 5 which are based on an oil-based gel.

### Experimental Example 1

The compositions of Examples 1 to 5 and Comparative Examples 1 to 2 above were used by 20 professional panelists to conduct a sensory evaluation of the fragrance persistence, and the results are shown in Table 2 below. The result value is a fragrance persistence time, and the average value of the values evaluated 3 times by 20 professional panelists who took 0.5 g of Examples 1 to 5 and Comparative Examples 1 to 2 and applied them to a 2 square centimeter area of the wrist.

**[Table 2] - Result value: Fragrance persistence time**

| Panel | | Examp.1 | Examp.2 | Examp.3 | Examp.4 | Examp.5 | Comparati ve Examp.1 | Comparati ve Examp.2 |
|---|---|---|---|---|---|---|---|---|
| | 1 | 14 | 13 | 13 | 14 | 10 | 11 | 11 |
| | 2 | 12 | 13 | 14 | 14 | 11 | 11 | 10 |
| | 3 | 14 | 14 | 13 | 14 | 11 | 12 | 11 |
| | 4 | 14 | 13 | 13 | 13 | 10 | 11 | 11 |
| Male | 5 | 12 | 13 | 12 | 13 | 12 | 12 | 9 |
| | 6 | 13 | 12 | 13 | 13 | 11 | 11 | 10 |
| | 7 | 13 | 13 | 12 | 13 | 10 | 11 | 11 |
| | 8 | 13 | 12 | 13 | 12 | 11 | 12 | 12 |
| | 9 | 14 | 12 | 13 | 13 | 11 | 11 | 10 |
| | 10 | 13 | 14 | 14 | 14 | 10 | 11 | 11 |
| | 11 | 15 | 13 | 14 | 14 | 12 | 12 | 12 |
| | 12 | 13 | 13 | 13 | 14 | 12 | 12 | 11 |
| | 13 | 14 | 13 | 13 | 14 | 11 | 11 | 11 |
| | 14 | 13 | 13 | 14 | 14 | 12 | 11 | 10 |
| | 15 | 12 | 14 | 13 | 14 | 12 | 12 | 11 |
| Female | 16 | 14 | 12 | 13 | 13 | 10 | 11 | 11 |
| | 17 | 14 | 13 | 13 | 12 | 11 | 12 | 10 |
| | 18 | 13 | 14 | 13 | 13 | 11 | 10 | 11 |
| | 19 | 13 | 13 | 14 | 14 | 12 | 12 | 11 |
| | 20 | 14 | 13 | 13 | 14 | 10 | 11 | 10 |
| | Average | 13.35 | 13 | 13.15 | 13.45 | 11 | 11.35 | 10.7 |

As shown in Table 2 above, the results that Examples 1 to 4, which are oil gels using dextrin-based gelling agents, showed long fragrance persistence times were obtained. Comparative Example 1 also showed a slightly longer fragrance persistence time than Comparative Example 2, which is a general liquid perfume, but there was no significant difference. Example 4, which showed the longest fragrance persistence time, showed a persistence that was about 25% longer than Comparative Example 1.

Since Examples 1 to 4 showed almost similar results, the fragrance persistence time according to the oil gelling agent was verified once more, and the contents and results are summarized in Tables 3 and 4 below.

**[Table 3] Examples 7 to 11, and Comparative Example 3 to 4 for each oil gelling agent based on 15% fragrance**

| | Raw Material Name | Examp. 7 | Examp. 8 | Examp. 9 | Examp. 10 | Examp. 11 | Compar ative Examp. 3 | Compar ative Examp. 4 |
|---|---|---|---|---|---|---|---|---|
| Oil phase | Isononylisononate | 36.8 | 36.8 | 36.8 | 36.8 | 41 | | |
| | Cetylethylhexanoate | 36 | 36 | 36 | 36 | 41 | | |
| Water phase | Water | | | | | | 47.4 | |
| | Glycerin | | | | | | 2 | |
| | Ethanol | | | | | | 20 | |
| | Dipropyleneglycol | | | | | | 15 | |
| Oil gelling agent | Dextrin palmitate | 12.2 | | 6.1 | 6 | | | |
| | Dextrin palmitate / ethylhexanoate | | 12.2 | 6.1 | 6 | | | |
| | Dibutylethylhexanoylglu tamide | | | | 0.1 | 1.5 | | |
| | Dibutyllauroylglutamide | | | | 0.1 | 1.5 | | |
| | Acrylate/C10-30 alkyl acrylate crosspolymer | | | | | | 0.6 | |
| Fragran ce | Fragrance | 15 | 15 | 15 | 15 | 15 | 15 | |
| Compa rison | Eau de Perfume (Fragrance 15%) | | | | | | | 100 |

As shown in Table 3 above, Examples 7 to 11 of the present invention are oil gel compositions. Examples 7 to 11 follow Examples 1 to 5 and are examples using 15% of fragrance.

Examples 7 to 9 of the present invention are compositions using dextrin palmitate and dextrin palmitate/ethylhexanoate, which are dextrin gelling agents, as oil gelling agents, respectively or in equal amounts at a ratio of 1:1. Example 10 is an example in which dextrin palmitate and dextrin palmitate/ethylhexanoate were used in a 1:1 ratio as in Example 9 as an oil gelling agent, and the amino acid-based gelling agent was added, and a composition that the total content of the oil gelling agent was the same as in Examples 7 to 9. Example 11 used only the amino acid-based gelling agent as the oil gelling agent, but since amino acid-based gelling agents have different gel-forming abilities and characteristics from dextrin-based gelling agents, the total content of the oil gelling agent was set as an exception only in Example 11. Comparative Example 3 is an example in which an aqueous gel containing ethanol is used as a base, unlike Examples 7 to 11 which are based on an oil-based gel.

### Experimental Example 2

Similar to Experimental Example 2, the compositions of Examples 7 to 11 and Comparative Examples 3 to 4 above were used by 20 professional panelists to conduct a sensory evaluation of the fragrance persistence, and the results are shown in Table 4 below. The result value is a fragrance persistence time, and the average value of the values evaluated 3 times by 20 professional panelists who took 0.5 g of Examples 7 to 11 and Comparative Examples 3 to 4 and applied them to a 2 square centimeter area of the wrist.

**[Table 4] - Result value: Fragrance persistence time**

| Panel | | Examp.7 | Examp.8 | Examp.9 | Examp.10 | Examp.11 | Comparati ve Examp.3 | Comparati ve Examp.4 |
|---|---|---|---|---|---|---|---|---|
| | 1 | 9 | 9 | 10 | 10 | 8 | 9 | 7 |
| | 2 | 8 | 8 | 8 | 9 | 7 | 8 | 6 |
| | 3 | 10 | 9 | 9 | 9 | 8 | 8 | 8 |
| | 4 | 10 | 8 | 9 | 9 | 8 | 9 | 8 |
| | 5 | 9 | 9 | 8 | 9 | 8 | 9 | 6 |
| Male | 6 | 9 | 10 | 9 | 10 | 8 | 9 | 7 |
| | 7 | 9 | 10 | 9 | 10 | 9 | 9 | 7 |
| | 8 | 9 | 9 | 9 | 9 | 8 | 8 | 7 |
| | 9 | 10 | 9 | 9 | 9 | 8 | 8 | 8 |
| | 10 | 9 | 9 | 8 | 8 | 7 | 8 | 6 |
| Female | 11 | 10 | 10 | 9 | 10 | 8 | 9 | 8 |
| | 12 | 10 | 9 | 10 | 9 | 8 | 8 | 7 |
| | 13 | 9 | 10 | 10 | 9 | 9 | 9 | 6 |
| | 14 | 9 | 8 | 9 | 9 | 7 | 8 | 7 |
| | 15 | 8 | 9 | 9 | 10 | 9 | 9 | 7 |
| | 16 | 9 | 9 | 10 | 9 | 8 | 8 | 7 |
| | 17 | 9 | 9 | 9 | 9 | 8 | 9 | 7 |
| | 18 | 10 | 10 | 9 | 10 | 9 | 8 | 8 |
| | 19 | 9 | 9 | 9 | 9 | 8 | 8 | 7 |
| | 20 | 8 | 8 | 9 | 9 | 7 | 7 | 6 |
| | Average | 9.15 | 9.05 | 9.05 | 9.25 | 8 | 8.4 | 7 |

As shown in Table 4 above, the results that Examples 7 to 10, which are oil gels using dextrin-based gelling agents, showed long fragrance persistence times were obtained. Comparative Example 3 also showed a slightly longer fragrance persistence time than Comparative Example 4, which is a general liquid perfume, but there was no significant difference. The results in Table 4 and Table 2 show almost similar trends. In particular, the fragrance persistence times of Examples 4 and 10 are the longest in common, which indicates that the combination of the dextrin-based gelling agent and the amino acid-based gelling agent contributes to the increase in fragrance persistence time. In Table 4, the fragrance persistence time of Example 10 lasted about 30% longer than that of Comparative Example 4.

The above description is merely an exemplary description of the technical idea of the present invention, and those with ordinary knowledge in the technical field to which the present invention pertains may make various modifications and variations without departing from the essential characteristics of the present invention.

Therefore, the embodiments disclosed in the present invention are not intended to limit the technical idea of the present invention but to explain it, and the scope of the technical idea of the present invention is not limited by these embodiments. The protection scope of the present invention should be interpreted by the claims below, and all technical ideas within the equivalent scope should be interpreted as being included in the scope of the rights of the present invention.

## Claims

1. A perfume gel composition comprising a fragrance, an oil, and an oil gelling agent, wherein the composition has a gel formulation for application to a skin.

2. The perfume gel composition according to claim 1,
wherein the perfume gel composition is substantially free of alcohol or water.

3. The perfume gel composition according to claim 1,
wherein the oil gelling agent comprises a dextrin-based oil gelling agent.

4. The perfume gel composition according to claim 3,
wherein the dextrin-based oil gelling agent is dissolved in an oil at a temperature of 60°C or higher.

5. The perfume gel composition according to claim 3,
wherein the dextrin-based oil gelling agent is a dextrin fatty acid ester.

6. The perfume gel composition according to claim 5,
wherein the fatty acid in the dextrin fatty acid ester comprises a saturated fatty acid having 8 to 30 carbon atoms.

7. The perfume gel composition according to claim 6,
wherein the dextrin fatty acid ester further comprises a substituted or unsubstituted C3-10 acid.

8. The perfume gel composition according to claim 7,
wherein the substitution is a substitution with a C1-6 alkyl.

9. The perfume gel composition according to claim 8,
wherein the dextrin fatty acid ester is dextrin palmitate, dextrin palmitate/ethylhexanoate, or a mixture thereof.

10. The perfume gel composition according to claim 3,
wherein the oil gelling agent further comprises an amino acid-based oil gelling agent.

11. The perfume gel composition according to claim 10,
wherein the amino acid-based oil gelling agent is a glutamide-based gelling agent.

12. The perfume gel composition according to claim 11,
wherein the amino acid-based gelling agent is dibutylethylhexanoylglutamide, dibutyllauroylglutamide, or a mixture thereof.

13. The perfume gel composition according to claim 1,
wherein the oil gelling agent is contained in an amount of 0.01 to 30% by weight, based on the total weight of the composition.

14. The perfume gel composition according to claim 1,
wherein a weight ratio of the oil and oil gelling agent is 2~ 99:1.

15. The perfume gel composition according to claim 10,
wherein a weight ratio of the dextrin-based oil gelling agent and the amino acid-based oil gelling agent is 100 : 0.1 to 50.

16. A preparation for external use to a skin comprising the perfume gel composition according to any one of claims 1 to 15.

17. A cosmetic composition comprising the perfume gel composition according to any one of claims 1 to 15.

18. A method for producing the perfume gel composition according to any one of claims 1 to 15, comprising:
adding the oil gelling agent to the oil and dissolving the mixture at a temperature of 60 to 160°C.
